# EUROPEAN PATENT APPLICATION

(11) **EP 4 821 422 A1**
(43) Date of publication of application: **14.10.2026**
(21) Application number: 25169109.3
(22) Date of filing: 08.04.2025
(51) Int. Cl.: A61B 1/00, A61B 3/10, A61B 3/12, A61B 3/13

(54) **AN OPHTHALMIC IMAGING DEVICE AND AN ABERRATION CORRECTION METHOD FOR AN OPHTHALMIC IMAGING DEVICE**

(71) Applicant: Optos plc, Dunfermline, Scotland KY11 8GR (GB)
(72) Inventor: Mizuta, Masahiro, Tokyo, 108-6290 (JP)
(74) Representative: Hoffmann Eitle

(57) **Abstract**

An ophthalmic device including a scanner configured to scan light from a light source in a specific direction, and an elliptical mirror configured to reflect the light scanned by the scanner, which is provided at a first focal point, toward a second focal point at which an examined eye is positioned. The ophthalmic device includes an optical element that is arranged on a light path between the light source and the scanner, that is configured to condense one part of the light at a first position on the light path, and that is configured to condense another part of the light at a second position on the light path.

## Description

### FIELD

The present disclosure generally relates to an ophthalmic imaging device, and to an aberration correction method for an ophthalmic imaging device. In particular, but not exclusively, it relates to correcting and/or suppressing aberrations in an ophthalmic imaging device.

### BACKGROUND

Ophthalmic imaging devices, such as scanning laser ophthalmoscopes (SLO), are used by clinicians to capture ophthalmic images of a patient's eye. For example, an ophthalmic imaging device may be used to acquire a fundus image of a patient's retina. A clinician may then review the fundus image to assess the ocular health of the patient.

Ophthalmic imaging devices may be wide-field or ultra-wide field of view imaging devices that can acquire wide-field or ultra-wide field fundus images. The Optos Daytona (RTM) is an example of an ultra-wide field ophthalmic imaging device that is arranged to acquire ultra-wide field fundus images of a patient's retina. Such ophthalmic imaging devices generally comprise a light source for outputting an imaging beam for imaging the patient's retina. The imaging beam is conveyed to and from the patient's eye by an optical system comprising a combination of optical elements and scan elements. In wide-field or ultra-wide field of view ophthalmic imaging devices the optical system that conveys the imaging beam to and from the patient's eye may comprise one or more concave or ellipsoidal mirrors. An example of an ultra-wide field mirror based ophthalmic imaging system is described in US 7959290.

Mirror based ophthalmic imaging systems beneficially allow clinicians to capture ultra-wide field of view fundus images with a field of view in the region of 200°. However, the curvature of the mirrors used in mirror-based ophthalmic imaging systems can introduce aberrations to the imaging beam which can negatively impact the quality of ophthalmic images captured by the mirror-based ophthalmic imaging system.

US 10149612 describes one way in which aberrations can be corrected in an ophthalmic imaging system by modifying the phase of the imaging light beam to provide correction of aberrations introduced by the mirrors in the ophthalmic imaging system.

It is an aim of the present invention to simplify the correction of aberrations in ophthalmic imaging systems, especially in ultra-wide field imaging systems that use ellipsoidal mirrors to convey light to and from a patient's eye.

### SUMMARY

According to an aspect of the present invention there is provided an ophthalmic imaging device for imaging a patient's eye, the ophthalmic imaging device comprising: a light source arranged to emit a beam of light along a light path; a scanner configured to scan the beam of light emitted from the light source; an elliptical mirror comprising a first focal point and a second focal point, wherein: during imaging the patient's eye is located at the second focal point of the elliptical mirror; and the scanner is arranged to scan the beam of light across a region of the patient's eye via the first focal point; and an optical element for correcting aberrations located on the light path between the light source and the scanner, wherein the optical element is configured to focus a first portion of the beam of light at a first focal position on the light path, and to simultaneously focus a second portion of the beam of light at a second focal position on the light path, wherein the first focal position is located at a different position to the second focal position on the light path.

The optical element is arranged to simultaneously focus a plurality of portions of the light beam at a plurality of different focal positions. Each of the focal positions may correspond to an aberration that the elliptical mirror imparts on the beam of light at a respective scan angle such that for each scan angle at least a portion of the imaging light beam is focused on the region of the patient's eye that is being imaged. The aberration may be, for example, a de-focus aberration imparted on the beam of light by the elliptical mirror.

The first and second focal positions may be located on the light path between the scanner and the elliptical mirror. The first and second focal positions may correspond to a focal length of the elliptical mirror at respective first and second scan angles of the beam of light such that at least a portion of light delivered to the patient's eye is substantially collimated light when the scanner is at the first and second scan angles.

The optical element may comprise a first region having a first refractive power and a second region having a second refractive power. Light passing through the first region of the optical element may be focused to the first focal position and light passing through the second region of the optical element may be focused to the second focal position. The optical element may comprise further regions each having distinct refractive powers such that light passing through each region is focused on a distinct focal point on the beam path.

The elliptical mirror may be arranged to reflect the first portion of the beam of light focused at the first focal position such that the first portion of the beam of light is substantially parallel light at the second focal point of the elliptical mirror. The light focused at the first focal position may be substantially parallel light at the second focal point when the scanner is orientated at a first scan angle. When the scanner is orientated at the first scan angle the second portion of the beam of light may be converging or diverging light at the second focal point. For example, the second portion of light may be de-focused light when the scanner is at the first scan angle.

The optical element may be a lens formed with rotational symmetry about an optical axis of the lens. The optical element may be a lens comprising a plurality of refraction regions each having a predetermined refractive power so as to provide a plurality of focal lengths for focusing the beam of light incident on the optical element.

The ophthalmic imaging device may further comprise an aperture located at a position that is conjugate with a pupil of the patient's eye, wherein the aperture is arranged to transmit focused light that has been reflected by the retina. The aperture may be arranged to block or discard de-focused light that has been reflected by the retina. The aperture may be a pin-hole aperture located between the scanner and a detector. The aperture may be arranged to transmit focused light that has been reflected by the retina such that the detector may detect the focused light and generate an image of the retina based on the detected focused light.

The ophthalmic imaging device may further comprise a second elliptical mirror. The second elliptical mirror may be orientated such that a reflection face of the elliptical mirror is opposed to a reflection face of the second elliptical mirror.

The ophthalmic imaging device may comprise a correction element that is arranged on a light path between the scanner and the patient's eye. The correction element may be configured to correct incident light such that light scanned by the scanner is irradiated onto the examined eye as parallel light. The correction element may be a phase mask for suppressing aberrations in the light beam.

The optical element may be dimensioned such that the beam of light illuminates the majority of the optical element and such that a major portion of light reflected by the region of the patient's eye does not pass through the optical element.

According to a further aspect of the present invention there is provided a method of correcting aberrations in an ophthalmic imaging device, the ophthalmic imaging device comprising an elliptical mirror and a scanner, the method comprising: emitting a beam of light along a light path; focusing the beam of light at two or more focal positions simultaneously to compensate for an aberration that the elliptical mirror imparts on the beam of light when the beam of light is incident on the elliptical mirror, wherein focusing the beam of light comprises focusing a first portion of the beam of light at a first focal position on the light path, and simultaneously focusing a second portion of the beam of light at a second focal position on the light path, wherein the first focal position is located at a different position to the second focal position on the light path; and scanning the focused beam of light across a region of the patient's eye via the elliptical mirror.

The aberration may be a de-focus aberration. The de-focus aberration may be imparted on the beam of light by the elliptical mirror. Beneficially, the method allows aberrations to be corrected or suppressed such that images acquired by an ophthalmic imaging system have less of the aberration. The method may comprise focusing a first portion of the beam of light at a first focal position and focusing a second portion of the beam of light at a second focal position. The first and second focal positions may be at different locations on the beam path. The first and second focal positions may be located between the scanner and the elliptical mirror.

Each of the two or more focal positions may correspond to an aberration that the elliptical mirror imparts on the beam of light at respective scan angles of the scanner. Focusing the beam of light may be performed at a location in the ophthalmic imaging device where the beam of light is stationary. For example, focusing the beam of light may be performed between a light source and the scanner within the ophthalmic imaging system.

The method may comprise irradiating the patient's eye with the beam of light wherein a first portion of the beam of light is substantially collimated light and wherein a second portion of the beam of light is converging or diverging light. The method may further comprise blocking the second portion of the beam of light after the second portion of the beam of light has been reflected by the patient's eye to prevent the second portion of the beam of light irradiating a detector. The first portion of the beam of light may be passed or transmitted through an aperture.

According to a further example aspect of the present invention there is provided an ophthalmic device, including: a scanner configured to scan light from a light source in a specific direction; an elliptical mirror configured to reflect the light scanned by the scanner, which is provided at a first focal point, toward a second focal point at which an examined eye is positioned; and an optical element that is arranged on a light path between the light source and the scanner, that is configured to condense one part of the light at a first position on the light path, and that is configured to condense another part of the light at a second position on the light path.

According to a yet further example aspect of the present invention there is provided an aberration correction method for an ophthalmic device that comprises a scanner that scans light from a light source in a specific direction, and an elliptical mirror that reflects the light scanned by the scanner, which is provided at a first focal point, toward a second focal point at which an examined eye is positioned. The aberration correction method includes arranging, on a light path between the light source and the scanner, an optical element that condenses one part of the light at a first position on the light path and condenses another part of the light at a second position on the light path, and performing aberration correction.

### BRIEF DESCRIPTION OF THE DRAWINGS

Exemplary embodiments of the present disclosure will now be described, by way of example only, based on the accompanying drawings, in which:
Figure 1 is a schematic configuration diagram of an ophthalmic system according to an exemplary embodiment;
Figure 2 is a schematic configuration diagram of an ophthalmic device according to an exemplary embodiment;
Figure 3 is a schematic configuration diagram of a server;
Figure 4 is an explanatory diagram of functions implemented by an image processing program in a CPU of a server;
Figure 5 is a schematic configuration diagram of an imaging optical system including a wide-angle optical system;
Figure 6 is a schematic configuration diagram of an imaging optical system including a wide-angle optical system;
Figure 7 is a schematic configuration diagram of scanning compensation lenses;
Figure 8 is an explanatory diagram related to a differential according to a scan angle of a scanning compensation lens;
Figure 9 is a schematic illustration of a light beam passing through a scanning compensation lens; and
Figure 10 is a flow chart showing a method of correcting aberrations in an ophthalmic imaging device.

### DETAILED DESCRIPTION

In general terms, embodiments of the invention relate to an ophthalmic imaging device comprising an optical element, such as a multi-focal lens arranged to simultaneously focus respective portions of an input light beam at distinct focal positions or planes. In mirror-based ophthalmic imaging systems aberrations may vary as a function of scan angle. The optical element beneficially focuses the input light beam simultaneously at multiple focal positions such that at least a portion of the light beam is in focus across a range of scan angles of the ophthalmic imaging device thereby suppressing the effects of aberrations, such as de-focus aberrations, in images acquired by the ophthalmic imaging system.

Detailed explanation follows regarding exemplary embodiments to implement technology disclosed herein, with reference to the drawings. Note that the same reference numerals are appended throughout the drawings to features, elements and processing modules with the same operation, and performing the same function, and duplicate explanation thereof will be omitted as appropriate. Moreover, sometimes description will be omitted for features not directly related to the technology disclosed herein and for known features. The dimensions and proportions in the drawings may be exaggerated for ease of explanation, and sometimes differ from actual proportions. Furthermore, each of the drawings are merely illustrated schematically to such a degree as to enable sufficient understanding of the technology disclosed herein. The technology disclosed herein is accordingly not limited to the examples illustrated.

The technology disclosed herein is applicable to any ophthalmic imaging device according to the technology disclosed herein. The ophthalmic imaging device may be an imaging device for acquiring images related to eyes, such as fundus images, optical coherence tomography (OCT) images or the like. In order to simplify the following explanation, the present exemplary embodiments will be described in the context of: an ophthalmic imaging device arranged to acquire ophthalmic images, and/or to an ophthalmic imaging device for an observer such as a clinician to observe an eye of a patient or the like (hereafter referred to as an examined eye). For example, the clinician may observe the examined eye to view the fundus of the eye, the periphery of the retina, or an anterior eye portion. The clinician may observe the eye for the purpose of assessing the ocular health of the patient, for the purpose of eye diagnostics in ophthalmology and/or for the purpose of surgical procedures to the eye.

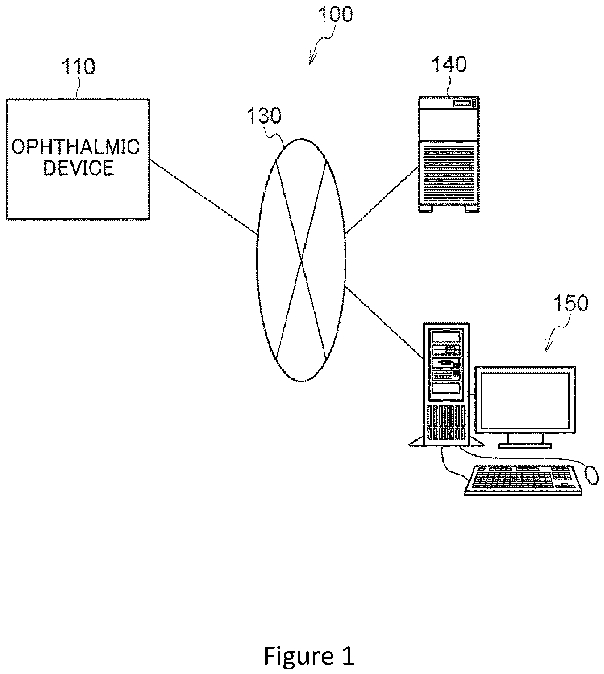

Figure 1 is a diagram illustrating a schematic configuration of an ophthalmic imaging system 100. As illustrated in Figure 1, the ophthalmic imaging system 100 includes an ophthalmic imaging device 110, a server device (hereafter referred to as "server") 140, and a display device (hereafter referred to as "viewer") 150. In an embodiment, the ophthalmic device 110 may be a scanning laser ophthalmoscope (SLO) arranged to an ophthalmic image of a patient's eye such as a fundus image. The server 140 may comprise memory arranged to store ophthalmic images of patients' eyes captured by the ophthalmic imaging device 110. The viewer 150 may be arranged to display the ophthalmic images that are captured by the ophthalmic imaging device 11 and stored on the server 140. The server 140 illustrates an example of an "image processing device" of the present disclosure.

The ophthalmic imaging device 110, the server 140, and the viewer 150 are connected together through a network 130. The network 130 may be a freely selected network such as a LAN, WAN, the internet, a wide area Ethernet, or the like. For example, a LAN may be employed as the network 130 in cases in which the ophthalmic system 100 is built in a single hospital.

The viewer 150 is a client in a client-server system, and one or more such devices may be connected together through the network 130. There may also be one or more servers 140 connected through the network in order to provide system redundancy. Alternatively, if the ophthalmic imaging device 110 is provided with image processing functionality and with the image viewing functionality of the viewer 150, then the ophthalmic images may be acquired and image processing and image viewing may be performed by the ophthalmic imaging device 110 in a standalone state. Moreover, if the server 140 is provided with the image viewing functionality of the viewer 150, then the ophthalmic images may be acquired and image processing and image viewing may be performed by a combination of the ophthalmic imaging device 110 and the server 140.

Note that other ophthalmic equipment (examination equipment for measuring a field of view, measuring intraocular pressure, or the like) and/or a diagnostic support device that analyzes images using artificial intelligence (AI) may be connected to the ophthalmic device 110, the server 140, and the viewer 150 over the network 130.

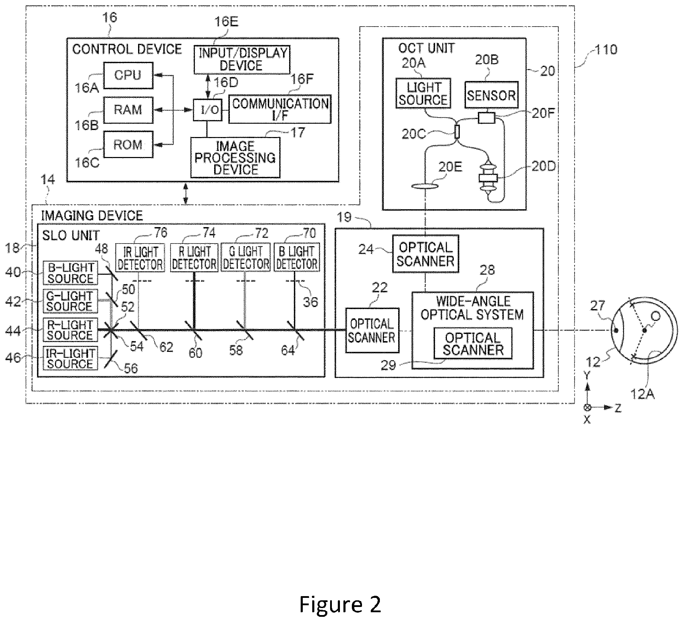

Next, explanation follows regarding an example configuration of the ophthalmic imaging device 110, with reference to Figure 2. For ease of explanation, scanning laser ophthalmoscope is abbreviated to SLO. Moreover, optical coherence tomography is abbreviated to OCT.

With the ophthalmic imaging device 110 installed on a horizontal plane and a horizontal direction taken as an "X direction", a direction perpendicular to the horizontal plane (a vertical direction) is denoted a "Y direction", and a direction connecting the center of the pupil at the anterior eye portion of the examined eye 12 and the center of the eyeball is denoted a "Z direction". The X direction, the Y direction, and the Z direction are mutually orthogonal. The X direction and the Y direction may be determined with reference to the above Z direction.

The ophthalmic imaging device 110 comprises an imaging device 14 and a control device 16 coupled to the imaging device 14. As shown in Figure 2, the imaging device 14 comprises an SLO unit 18, and an OCT unit 20. The SLO unit 18 and OCT unit 20 are arranged to acquire ophthalmic images of the examined eye 12. Two-dimensional fundus images that have been acquired by the SLO unit 18 are referred to below as SLO images. Tomographic images, face-on images (en face images) and the like of the retina or anterior chamber created based on OCT data acquired by the OCT unit 20 are referred to as OCT images. Note that the imaging device 14 of the ophthalmic imaging device 110 may be a device provided with either the SLO unit 18 or OCT unit 20 alone.

The control device 16 may comprise a computer provided with a Central Processing Unit (CPU) 16A, Random Access Memory (RAM) 16B, Read-Only Memory (ROM) 16C, and an input/output (I/O) port 16D.

The control device 16 may also comprise an input/display device 16E connected to the CPU 16A through the I/O port 16D. The input/display device 16E may include a user interface such as a graphical user interface (not shown) to display images of the examined eye 12 and to receive various inputs from a user, such as a clinician. An example of the graphical user interface is a touch panel display.

The control device 16 may also comprise an image processing device 17 connected to the I/O port 16D. The image processing device 17 is configured to generate ophthalmic images of the examined eye 12 based on data acquired by the imaging device 14. Note that the control device 16 may be connected to the network 130 through a communication interface (I/F) 16F. Note also that the image processing device 17 may include non-illustrated memory. The non-illustrated memory of the image processing device 17 may be employed as memory to store various data under control of the control device 16.

Although, as described above, the control device 16 of the ophthalmic imaging device 110 is provided with the input/display device 16E as illustrated in Figure 2, the embodiments disclosed herein not limited thereto. For example, a configuration may be implemented in which the control device 16 of the ophthalmic device 110 is not provided with the input/display device 16E, and instead a separate input/display device is provided that is physically independent of the ophthalmic imaging device 110. In such cases, the display device is provided with an image processing processor unit that operates under the control of a display control section 204 of the CPU 16A in the control device 16. Such an image processing processor unit may be configured so as to display SLO images and/or OCT images or the like based on image signals output as instructed by the display control section 204.

As shown in Figure 2, the imaging device 14 is coupled to the control device 16 such that the imaging device 14 can operate under the control of the control device 16 and, in particular, under the control of the CPU 16A of the control device 16. The imaging device 14 may include the SLO unit 18 and/or the OCT unit 20, and an imaging optical system 19. The imaging optical system 19 may include one or more of optical scanners 22, 24, and a wide-angle or ultra-wide angle optical system 28. The wide-angle or ultra-wide angle optical system 28 may comprise an optical scanner 29.

The optical scanner 22 is arranged to scan a beam of light emitted from the SLO unit 18 in a specific direction across a region of the patient's eye 12 to image the patient's eye 12. The optical scanner 22 is an optical element capable of scanning or deflecting light beams. The optical scanner 22 may be, for example, any one of: a polygon scanning mirror, a scanning mirror galvanometer, or any other scanning system applicable to ophthalmic imaging. A combination thereof may also be employed. The optical scanner 29 may scan incident light in a direction intersecting with the specific direction. As long as the optical scanner 29 is an optical element capable of deflecting light beams, it may be configured by any out of, for example, a polygon mirror, a mirror galvanometer, or the like. A combination thereof may also be employed. The optical scanners 22, 29 accordingly perform two-dimensional scanning of light emitted from the SLO unit 18, in the X direction and the Y direction.

The optical scanner 24 scans light emitted from the OCT unit 20 in a specific direction across a region of the patient's eye 12 to image the patient's eye 12. The optical scanner 24 is an optical element capable of scanning or deflecting light beams. The optical scanner may be, for example, any one of: a polygon scanning mirror, a scanning mirror galvanometer, or any other scanning system applicable to ophthalmic imaging. A combination thereof may also be employed. The optical scanners 24, 29 accordingly scan light emitted from the OCT unit 20 two dimensionally, in the X direction and the Y direction.

Note that the optical scanners 22, 24 are not limited to being configured by different optical scanners, and a common optical scanner may be employed therefor.

Moreover, the wide-angle or ultra-wide angle optical system 28 guides light from the SLO unit 18 and light from the OCT unit 20 to the examined eye. Detailed explanation follows regarding the imaging optical system 19 including the wide-angle optical system 28.

Note that the wide-angle optical system 28 may be a reflection optical system employing one or more concave mirrors such as a second reflection mirror, a refraction optical system employing a wide-angle lens, or may be a reflection-refraction optical system employing a combination of a concave mirror and a lens. Employing a wide-angle optical system that utilizes a second reflection mirror, wide-angle lens, or the like enables imaging to be performed not only of a central portion of the fundus, but also of the retina at the fundus periphery.

For a system including a second reflection mirror, a configuration may be adopted that utilizes a second reflection mirror system as disclosed in International Publication (WO) Nos. 2016/103484 or 2016/103489. The disclosures of WO Nos. 2016/103484 and 2016/103489 are incorporated in their entirety in the present specific by reference herein.

Observation of the fundus over a wide field of view (FOV) 12A may be implemented by the wide-angle optical system 28. The FOV 12A refers to a range capable of being imaged by the imaging device 14. The FOV 12A may be expressed as a viewing angle. In the present exemplary embodiment the viewing angle may be defined in terms of an internal illumination angle and an external illumination angle. The external illumination angle is the angle of illumination by a light beam illuminated from the ophthalmic device 110 toward the examined eye 12, and is an angle of illumination defined with respect to a pupil 27. The internal illumination angle is the angle of illumination of a light beam illuminated onto the fundus, and is an angle of illumination defined with respect to an eyeball center O. Correspondence relationships exist between the external illumination angle and the internal illumination angle. The internal illumination angle in the present exemplary embodiment may be approximately 200°.

SLO fundus images obtained by imaging at an imaging angle of view having an internal illumination angle of 160° or greater are referred to as UWF-SLO fundus images. UWF is an abbreviation for ultra-wide field (ultra-wide angled). A region extending from a posterior pole portion of a fundus of the examined eye 12 past an equatorial portion thereof can be imaged by the wide-angle optical system 28 having a field of view (FOV) angle of the fundus that is an ultra-wide field, enabling imaging of structural objects, such as vortex veins, present at fundus peripheral portions.

The ophthalmic imaging device 110 may be capable of imaging a region 12A with an internal illumination angle of up to approximately 200° with respect to the eyeball center O of the examined eye 12 as a reference position.

An example SLO system may comprise the control device 16, the SLO unit 18, and the imaging optical system 19 as illustrated in Figure 2. The SLO system is provided with the wide-angle optical system 28, enabling fundus imaging over the wide FOV 12A. The skilled reader would understand that in an embodiment that did not comprise the OCT unit 20 then optical scanner 24 may be omitted. Similarly, in an embodiment that did not comprise the SLO unit 18 then optical scanner 22 may be omitted.

The SLO unit 18 may be provided with one or more of a blue (B) light source 40, a green (G) light source 42, a red (R) light source 44, an infrared (for example near infrared) (IR) light source 46, and optical systems 48, 50, 52, 54, 56 to guide the light from the light sources 40, 42, 44, 46 onto a single light path using reflection and/or transmission. In the present example embodiment, the optical systems 48, 56 are mirrors, and the optical systems 50, 52, 54 are beam splitters. B light is reflected by the optical system 48, is transmitted through the optical system 50, and is reflected by the optical system 54. G light is reflected by the optical systems 50, 54, R light is transmitted through the optical systems 52, 54, and IR light is reflected by the optical systems 52, 56. The respective lights are thereby guided onto a single light path.

The SLO unit 18 is configured so as to be capable of switching between light sources for emitting laser light of different wavelengths or a combination of light-emitting light sources, such as a mode in which R light and G light are emitted, a mode in which infrared light is emitted, etc. Although the example in Figure 2 includes four light sources, i.e. the B light source 40, the G light source 42, the R light source 44, and the IR light source 46, the present disclosure is not limited thereto. For example, the SLO unit 18 may further include a white light source, in a configuration in which light is emitted in various modes, such as a mode in which G light, R light, and B light are emitted, and a mode in which white light is emitted alone.

Incident light to the imaging optical system 19 from the SLO unit 18 is scanned in the X direction and the Y direction by the optical scanner 22, 29. The scanning light passes through the wide-angle optical system 28 and the pupil 27 and is illuminated onto the fundus. Reflected light that has been reflected by the fundus passes through the wide-angle optical system 28 and the optical scanner 22 and is incident to the SLO unit 18.

The SLO unit 18 is provided with a beam splitter 64 and a beam splitter 58. From out of the light coming from a posterior eye portion (fundus) of the examined eye 12, the B light therein is reflected by the beam splitter 64 and light other than B light therein is transmitted by the beam splitter 64. From out of the light transmitted by the beam splitter 64, the G light therein is reflected by the beam splitter 58 and light other than G light therein is transmitted by the beam splitter 58. The SLO unit 18 is further provided with a beam splitter 60 that, from out of the light transmitted through the beam splitter 58, reflects R light therein and transmits light other than R light therein. The SLO unit 18 is further provided with a beam splitter 62 that reflects IR light from out of the light transmitted through the beam splitter 60. The SLO unit 18 is further provided with a B light detector 70 to detect B light reflected by the beam splitter 64, a G light detector 72 to detect G light reflected by the beam splitter 58, an R light detector 74 to detect R light reflected by the beam splitter 60, and an IR light detector 76 to detect IR light reflected by the beam splitter 62.

Note that in order to separate light illuminated from a light source and light reflected from the examined eye, a reflection element may be applied for the beam splitter 58, 60, 62, 64 such as a hole mirror, a dot mirror, or the like provided with a hole at a part including a location where light of a specific wavelength is transmitted, with other locations thereof being locations where such light is reflected. For example, a hole mirror that transmits light from the B light source 40 and reflects reflected light from the examined eye may be applied as the beam splitter 64. The hole mirror employed in the beam splitter 64 may have a configuration in which a hole portion is disposed on the optical axis of B light so as to transmit light from the B light source 40.

Light that has passed through the wide-angle optical system 28 and the optical scanner 22 and is incident light to the SLO unit 18 (i.e. reflected light that has been reflected by the fundus) is reflected by the beam splitter 64 and photo-detected by the B light detector 70 when B light, and is transmitted through the beam splitter 64, reflected by the beam splitter 58, and photo-detected by the G light detector 72 when G light. The incident light when R light is transmitted through the beam splitters 64, 58, reflected by the beam splitter 60, and photo-detected by the R light detector 74. The incident light when IR light is transmitted through the beam splitters 64, 58, 60, reflected by the beam splitter 62, and photo-detected by the IR light detector 76. The image processing device 17 that operates under the control of the CPU 16A employs signals detected by the B light detector 70, the G light detector 72, the R light detector 74, and the IR light detector 76 to generate UWF-SLO images.

UWF-SLO images generated using signals detected by the B light detector 70 are called B-UWF-SLO images (blue fundus images). UWF-SLO images generated using signals detected by the G light detector 72 are called G-UWF-SLO images (green fundus images). UWF-SLO images generated using signals detected by the R light detector 74 are called R-UWF-SLO images (red fundus images). UWF-SLO images generated using signals detected by the IR light detector 76 are called IR-UWF-SLO images (IR fundus images). UWF-SLO images encompass the red fundus images, the green fundus images, the blue fundus images, and the IR fundus images. Florescent light UWF-SLO images imaged with florescent light are also encompassed therein.

The control device 16 may also control the light sources 40, 42, 44 such that one or more of the light sources 40, 42, 44 emit light simultaneously. A green fundus image, a red fundus image, and a blue fundus image are obtained with mutually corresponding positions by imaging the fundus of the examined eye 12 at the same time with the B light, G light, and R light. An RGB color fundus image is obtained from the green fundus image, the red fundus image, and the blue fundus image. The control device 16 obtains a green fundus image and a red fundus image with mutually corresponding positions by controlling the light sources 42, 44 so as to emit light at the same time and by imaging the fundus of the examined eye 12 at the same time with the G light and R light. An RG color fundus image is obtained from the green fundus image and the red fundus image. Moreover, a full color fundus image may be generated using the green fundus image, the red fundus image, and the blue fundus image.

A region extending from a posterior pole portion of a fundus of the examined eye 12 past an equatorial portion thereof can be imaged by the wide-angle optical system 28 with a field of view (FOV) angle of the fundus that is an ultra-wide field.

An OCT system may be implemented by the control device 16, the OCT unit 20, and the imaging optical system 19 illustrated in Figure 2. The OCT system includes the wide-angle optical system 28, and is accordingly able to perform OCT imaging of fundus peripheral portions similarly to the imaging of SLO fundus images described above. Namely, OCT imaging over a region extending from a posterior pole portion of the examined eye 12 fundus past the equatorial portion 178 is able to be performed by employing the wide-angle optical system 28 having a field of view (FOV) angle of the fundus that is an ultra-wide field. OCT data of structural objects such as vortex veins present in the fundus peripheral portions can be acquired, and tomographic images of vortex veins and a 3D structure of vortex veins can be obtained by performing image processing on the OCT data.

The OCT unit 20 shown in Figure 2 comprises a light source 20A, a sensor (detection element) 20B, a first light coupler 20C, a reference optical system 20D, a collimator lens 20E, and a second light coupler 20F.

Light emitted from the light source 20A is split by the first light coupler 20C. One part of the split light is collimated by the collimator lens 20E into parallel light serving as measurement light before being incident to the imaging optical system 19. The measurement light is illuminated onto the fundus through the wide-angle optical system 28 and the pupil 27. Measurement light that has been reflected by the fundus passes through the wide-angle optical system 28 so as to be incident to the OCT unit 20, then passes through the collimator lens 20E and the first light coupler 20C before being incident to the second light coupler 20F.

The other part of the light emitted from the light source 20A and split by the first light coupler 20C is incident to the reference optical system 20D as reference light, and is made incident to the second light coupler 20F through the reference optical system 20D.

The respective lights that are incident to the second light coupler 20F, namely the measurement light reflected by the fundus and the reference light, interfere with each other in the second light coupler 20F so as to generate interference light. The interference light is photo-detected by the sensor 20B. The image processing device 17 operating under the control of an image processing section 206 generates OCT data detected by the sensor 20B. OCT images, such as tomographic images and en face images, are generated in the image processing device 17 based on this OCT data.

The OCT unit 20 is able to scan a specific range (for example a rectangular range of 6 mm × 6 mm) at a single time of OCT imaging. The specific range is not limited to being 6 mm × 6 mm, and may be a square range of 12 mm × 12 mm or 23 mm × 23 mm, may be a rectangular range of 14 mm × 9 mm, 6 mm × 3.5 mm, or the like, and may be a freely selected rectangular range. Moreover, the specific range may be a circular range having a diameter of 6 mm, 12 mm, 23 mm, or the like.

By employing the wide-angle optical system 28, the ophthalmic device 110 is able to designate the region 12A having an internal illumination angle of 200° as the scan target. Namely, OCT imaging is performed of the specific range including vortex veins, for example, by controlling the optical scanners 24, 29. The ophthalmic device 110 is able to generate OCT data by this OCT imaging.

Thus, the ophthalmic device 110 is able to generate OCT images such as tomographic images of the fundus including vortex veins (B-scan images), OCT volume data including vortex veins, and en-face images that are cross-sections of such OCT volume data (face-on images generated based on the OCT volume data). It will be understood that the term "OCT images" also encompasses an OCT image of a fundus center portion (posterior pole portion of the eyeball where the macular, the optic nerve head, and the like are present).

The OCT data (or image data of the OCT images) may be sent from the ophthalmic device 110 to the server 140 though the communication interface 16F and is stored in a storage device 254. Note that although in the present exemplary embodiment an example is given in which the light source 20A is a wavelength swept-source OCT (SS-OCT), various types of OCT system may be employed, such as a spectral-domain OCT (SD-OCT) or a time-domain OCT (TD-OCT) system.

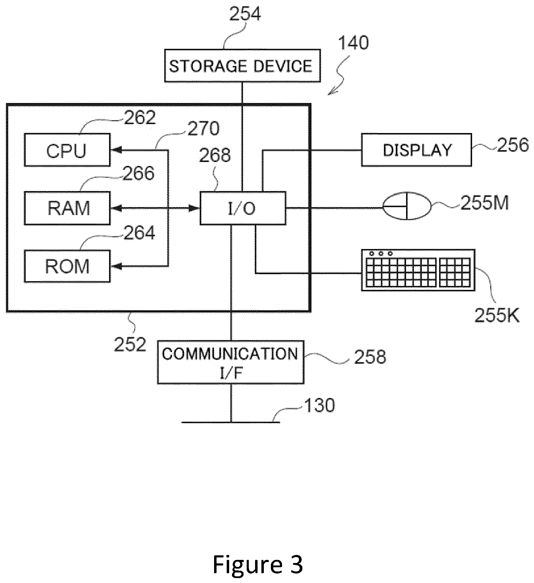

Next, description follows regarding a configuration of an electrical system of the server 140, with reference to Figure 3. As illustrated in Figure 3, the server 140 includes a computer main body 252. The computer main body 252 includes a CPU 262, RAM 266, ROM 264, and an input/output (I/O) port 268. The CPU 262, the RAM 266, the ROM 264, and the input/output port (I/O) 268 may be connected so as to be capable of communicating with each other through a bus 270. The storage device 254, a display 256, a mouse 255M, a keyboard 255K, and a communication interface (I/F) 258 are connected to the input/output (I/O) port 268. The storage device 254 is, for example, configured by non-volatile memory. The input/output (I/O) port 268 is connected to the network 130 through the communication interface (I/F) 258. The server 140 is accordingly able to communicate with the ophthalmic device 110 and the viewer 150.

An image processing program is stored in the ROM 264 or the storage device 254. The ROM 264 and the storage device 254 are examples of "memory" of the present disclosure. The CPU 262 is an example of a "processor" of the present disclosure. The image processing program is an example of a "program" of the present disclosure.

The server 140 stores respective data received from the ophthalmic device 110 in the storage device 254. Moreover, the server 140 is able to use the various data received to store data of a process related to image processing and processing result data in the storage device 254. Description follows regarding various functions implemented by the CPU 262 of the server 140 executing the image processing program.

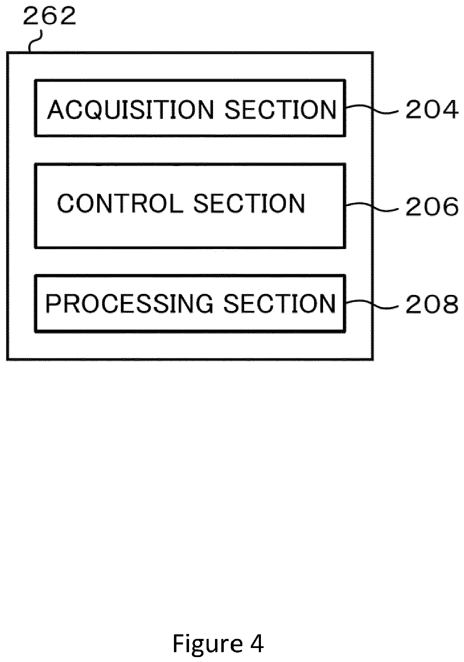

The image processing program executed in the CPU 262 may include respective sections to implement one or more of a display control function, an image processing function, and a processing function. Specifically, by the CPU 262 executing the image processing program, the CPU 262 operates, as illustrated in Figure 4, as the display control section 204 to implement a display control function, the image processing section 206 to implement an image processing function, and a processing section 208 to implement a processing function.

Next, description follows regarding image processing by the server 140. The CPU 262 of the server 140 implements image processing (an image processing method) by executing the image processing program.

First, by executing the image processing program, the CPU 262 performs processing in the image processing section 206 to acquire a fundus image from the storage device 254. This fundus image includes, for example, data related to blood vessels, such as choroidal vessels including vortex veins. Next, the CPU 262 performs processing in the image processing section 206 to execute various predetermined image processing on the acquired fundus image, such as emphasizing blood vessels in the fundus image, generating a three-dimensional image of choroidal vessels, and the like. Next, the CPU 262 performs processing in the processing section 208 to output an image generated after the image processing, specifically processing to save the image in the RAM 266 or the storage device 254, and then ends the image processing.

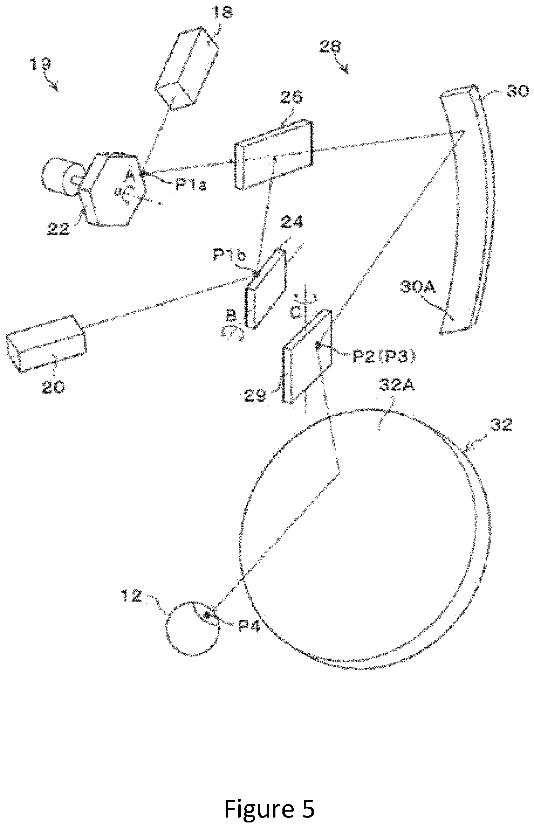

Explanation follows regarding the imaging optical system 19 included in the wide-angle optical system 28, with reference to Figure 5. The wide-angle optical system 28 may be configured to include a first reflection mirror 30 and a second reflection mirror 32, as illustrated in Figure 5. The first and second reflection mirrors 30, 32 may either or both be elliptical mirrors.

As shown in Figure 5, the optical scanner 22 may be, for example a polygon scanning mirror, arranged to scan light from the SLO unit 18 in the Y direction by rotating in the direction of arrow A. Moreover, the optical scanner 24, for example a mirror galvanometer, scans light from the OCT unit 20 in the Y direction by rotating in the direction of arrow B.

The wide-angle optical system 28 may include a mirror, for example a dichroic mirror 26, a first reflection mirror 30 having an elliptical concave reflection face, the optical scanner 29, for example a mirror galvanometer, and the second reflection mirror 32 having an elliptical concave reflection face.

The dichroic mirror 26 is arranged to transmit light emitted from the SLO unit 18 and to reflect light emitted from the OCT unit 20, thereby guiding both light beams onto a common light path that can propagate through the wide-angle optical system 28 to the patient's eye 12. As shown in Figure 5, light emitted from the SLO unit 18 is aligned with the common light path such that when the dichroic mirror 26 transmits the light emitted from the SLO unit 18 that light can travel along the common light path. The dichroic mirror 26 is orientated such that light emitted by the OCT unit 20 is reflected by the dichroic mirror 26 along the common light path towards the patient's eye 12. The skilled person will understand that the dichroic mirror 26 is an optional feature and, for example, in an ophthalmic imaging system with only SLO imaging functionality the dichroic mirror 26 may be omitted.

The dichroic mirror 26 is arranged to transmit light emitted from the SLO unit 18 and to reflect light emitted from the OCT unit 20, thereby guiding both light beams onto a common light path that can propagate through the wide-angle optical system 28 to the patient's eye 12. As shown in Figure 5, light emitted from the SLO unit 18 is aligned with the common light path such that when the dichroic mirror 26 transmits the light emitted from the SLO unit 18 that light can travel along the common light path. The dichroic mirror 26 is orientated such that light emitted by the OCT unit 20 is reflected by the dichroic mirror 26 along the common light path towards the patient's eye 12. The skilled person will understand that the dichroic mirror 26 is an optional feature and, for example, in an ophthalmic imaging system with only SLO imaging functionality the dichroic mirror 26 may be omitted.

The first reflection mirror 30 reflects the incident light to a mirror face 30A, and thereby guides this reflected light to the optical scanner 29. The optical scanner 29 scans incident light in the X direction by rotational oscillation in the directions of arrow C.

The first reflection mirror 30 and the second reflection mirror 32 are examples of an "elliptical mirror" of the present disclosure. Each of the elliptical mirrors comprise two focal points. The optical scanner 22 is located at a first focal point of the first mirror 30 and the optical scanner 29 is located at a second focal point of the first mirror 30. The second mirror is positioned such that the optical scanner 29 is also located at a first focal point of the second mirror 32 and a patient's pupil 27 is located at the second focal point of the second mirror 32 when imaging the patient's eye 12.

The second reflection mirror 32 reflects incident light to a mirror face 32A so as to guide this reflected light onto the fundus of the examined eye 12. Note that the illuminated light guided onto the fundus (retina) is reflected at the fundus and the reflected light propagates through the wide-angle optical system 28 via the common light path, to the detectors located in the SLO unit 18 and the OCT unit 20 such that an image of the fundus can be generated.

Th reflected light is guided through the wide-angle optical system 28 to the dichroic mirror 26 along the same light path to that of the light described above, but in the opposite direction. The dichroic mirror 26 guides the fundus reflected light arising from the light from the SLO unit 18 into the SLO unit 18, and guides the fundus reflected light arising from the light from the OCT unit 20 into the OCT unit 20.

The first elliptical mirror 30 and the second elliptical mirror 32 are examples of a "reflection mirror" of the present disclosure.

In the wide-angle optical system 28 described above, the optical scanner 22 and the optical scanner 24 are arranged such that a scanning central position of light from the SLO unit 18 in the optical scanner 22 is conjugate to the scanning central position of light from the optical scanner 24. Moreover, in the wide-angle optical system 28, the optical scanner 22, the optical scanner 24, and the optical scanner 29 are arranged such that a scanning central position of light from the optical scanner 29 is conjugate to the scanning central position of light from the optical scanner 22 and from the optical scanner 24. The pupil of the eyeball of the examined eye 12 is positioned at one of the focal positions of the second reflection mirror 32.

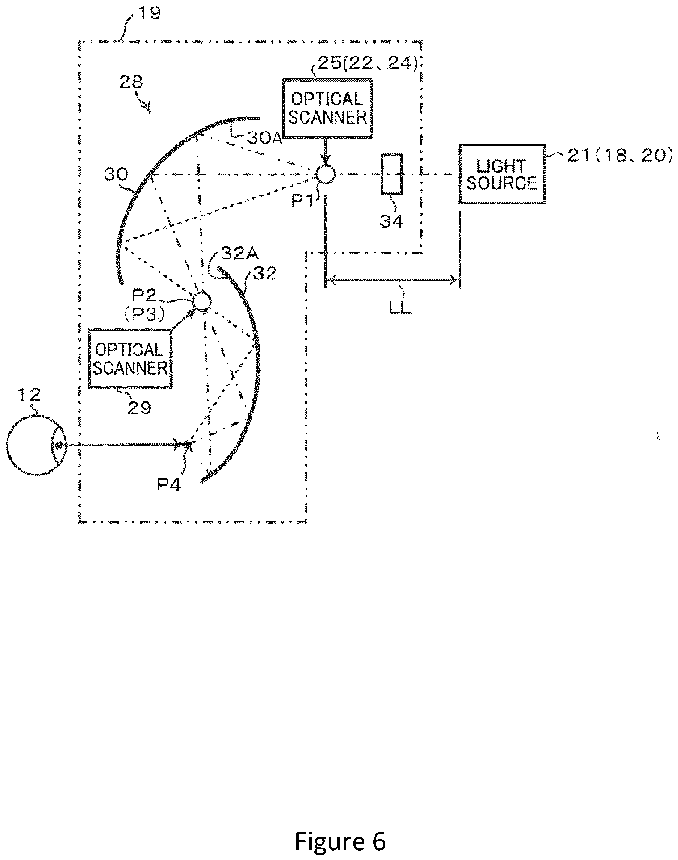

The optical scanner 29 is arranged such that a scanning central position of light of the optical scanner 29 is at a pupil conjugate position P3 conjugate to a pupil position P4 of the examined eye 12 formed by the second reflection mirror 32, this being the other focal position of the second reflection mirror 32. The first reflection mirror 30 and the optical scanner 29 are arranged such that one focal position of the first reflection mirror 30 having the elliptical concave reflection face is positioned at the scanning central position of light from the optical scanner 29. The pupil conjugate position P3 due to the second reflection mirror 32, and the pupil conjugate position P2 due to the first reflection mirror 30, are accordingly arranged so as to be a common position (see Figure 6). The optical scanners 22, 24 are arranged such that the scanning central position of light from the optical scanners 22, 24 is at the other focal position of the first reflection mirror 30, this being a pupil conjugate position P1 conjugate to the pupil conjugate position P2 formed by the first reflection mirror 30.

When scanning a fundus of the examined eye 12 or the like, for example, the light from light sources of the SLO unit 18 should be incident on the examined eye as a parallel light beam. This is because scanning the fundus with a parallel light beam yields the highest quality ophthalmic images of the fundus. However, in cases in which the wide-angle optical system 28 is configured to include the second reflection mirror 30, when light from the light sources of the SLO unit 18 is incident to the imaging optical system 19 as a parallel light beam, and is reflected at the first reflection mirror 30 and the second reflection mirror 32, this light is diffuse light at the position of the examined eye pupil irrespective of the scan angle. When the light at the position of the examined eye pupil is diffuse light, this light is refracted by the crystalline lens but is not condensed onto the retina. In contrast thereto, when light at the position of the examined eye 12 pupil is parallel light, then this light is refracted by the crystalline lens so as to be condensed onto the retina. This means that due to the light not being condensed onto the retina when diffuse light is incident to the examined eye, the amount of information obtained from the retina is less than when parallel light is incident to the examined eye thereby resulting in lower quality ophthalmic images.

However, if an optical element such as a lens or the like is inserted at the emission side of the light sources, and light is incident to the imaging optical system 19 as a convergent light beam and reflected by the first reflection mirror 30 and the second reflection mirror 32, then sometimes this results in state of light at the position of the examined eye pupil being any of diffuse light, converging light, or parallel light, depending on the scan angle of the light beam. For example, if the optical element is a lens system that is inserted such that a light beam at the scanning center of the optical scanner 22 or 24 is a parallel light beam at the position of the examined eye pupil, then some of the light away from the scanning center becomes converging light, and other light becomes diffuse light. This means that when light is incident to the imaging optical system 19 as a convergent light beam, although the amount of information obtained from the retina by the light beam at the scanning center of the optical scanner 22 or 24 is greater, the amount of information obtained from other light beams not at the scanning center is less. Note that although a case has been described in which the light beam at the scanning center of the optical scanner 22 or 24 is condensed onto the retina by the configuration of the lens system, there is no limitation thereto.

Thus in the present exemplary embodiment, an optical system is described such that when light from light sources is scanned onto the examined eye 12, the light from light sources is incident to the examined eye as parallel light for light at at least some scan angles of the scanner.

Next, description follows regarding an embodiment of the imaging optical system 19 including the wide-angle optical system 28, with reference to Figure 6.

The scanning central positions of light of the optical scanners 22 and 24 are conjugate in the wide-angle optical system 28 as described above, and are also at the pupil conjugate position P1, and so the SLO unit 18 and the OCT unit 20 are denoted as a light source 21 in Figure 6. The optical scanners 22, 24 are also denoted as an optical scanner 25 in Figure 6. The skilled reader will understand that in another embodiment the imaging device 19 of the ophthalmic imaging device 110 may only comprise one of the SLO unit 18 and the OCT unit 20. Furthermore, in an embodiment where only one of the SLO unit 18 and OCT unit 20 is present only one scanner 22, 24 would be present in the imaging optical system 19.

As illustrated in Figure 6, a scanning compensation lens 34 (also referred to as an optical element) used for scanning compensation and/or for correcting aberrations is arranged on a light path between the light source 21 and the scanner 25. The scanning compensation lens 34 is located before scanning in the imaging optical system 19, specifically on a light path LL between the light source 21 and the optical scanner 25. Locating the scanning compensation lens 34 before the optical scanner 25 means light from the light source 21 travelling along the light path LL towards the optical scanner 25 is stationary when it passes through the scanning compensation lens 34. The scanning compensation lens 34 is an example of an "optical element" of the present disclosure.

A single lens, or a lens optical system including multiple lenses, that converges light from the light source 21 at a plurality of different positions may form the scanning compensation lens 34 or optical element. The scanning compensation lens 34 is an optical element that, for the light scanned by either or both the optical scanner 25 and the optical scanner 29, corrects a state in which light converges at different positions depending on scan angle, such that parallel light is illuminated onto the examined eye. Namely, the scanning compensation lens 34 is an optical element arranged on the light path between the light source 21 and the optical scanner 25 before light from the light source 21 is scanned by the scanner 25. The scanning compensation lens 34 is arranged to receive a stationary light beam from the light source 21 and focus the received light at more than one location simultaneously. As such, different portions of the input light beam are simultaneously focused at different focal points each having a different focal length. The scanning compensation lens 34 makes the light from the light source 21 converge at a plurality of different positions, and thereby enables correction of aberration according to scan angle generated by light from the optical scanners 25, 29 being deflected and reflected at the first reflection mirror 30 and the second reflection mirror 32.

Moreover, light restricting elements 36 are arranged on the return light paths of the imaging device 14, for example, on the light paths of reflected light from the examined eye in the SLO unit 18. The light restricting elements 36 are each arranged at a fundus conjugate plane and for example, are arranged in the detection optical system at the light detector side in the SLO unit 18, and function as a confocal diaphragm in the imaging device 14. The light restricting elements 36 are each an optical member that restricts light passing along a light path, such as a pinhole member or the like formed with an aperture portion provide with a hole of a predetermined size. An optical system of the imaging device 14 is formed such that light from the fundus cross at a focal point at the aperture portion of the light restricting element 36. Light from positions other than a condensing spot (or focal plane) on the fundus is removed by the light restricting elements 36, and the light remaining after removal (light from the condensing spot) is guided to the respective light detector. For example, out of reflected B light from the examined eye, light from the condensing spot on the fundus passes through one of the light restricting elements 36 and is guided to the B light detector 70. Similarly, reflected G light is guided to the G light detector 72, reflected R light is guided to the R light detector 74, and reflected IR light is guided to the IR light detector 76. Note that the light restricting elements 36 may each be arranged at a pupil conjugate position of the examined eye, or at a position in the vicinity thereof.

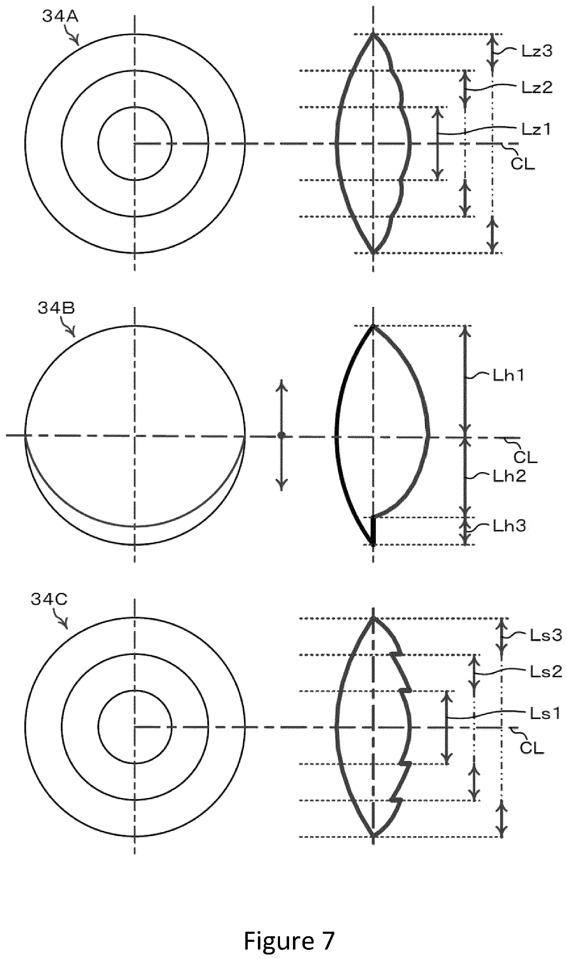

Next, description follows regarding example embodiments of the scanning compensation lens 34, with reference to Figure 7. Figure 7 illustrates the scanning compensation lenses 34A, 34B, 34C as examples of the scanning compensation lens 34 or optical element.

As illustrated in Figure 7, each of the scanning compensation lenses 34A, 34B, 34C comprise multiple regions each of which have a different focal length. Typically, the scanning compensation lenses 34A, 34B, 34C are dimensioned such that when the scanning compensation lens 34 is positioned on the light path between the light source 21 and the scanner 25 the light beam illuminates the majority of the scanning compensation lens 34. For example, the diameter of the light beam may be substantially equal to the diameter of the scanning compensation lens 34 or the diameter of the scanning compensation lens may be larger than the diameter of the light beam such that the light beam illuminates about 70% or more of the scanning compensation lens 34. In scanning laser ophthalmoscopes the return light to the SLO unit 18 has a larger diameter than the diameter of the input light beam. As such, in some embodiments the majority of the return light does not pass through the scanning compensation lens 34.

The scanning compensation lens 34A includes lens regions centered on the optical axis CL of lens components each giving predetermined focal lengths, with each of the lens regions converging or focusing light at a different position. Namely, the scanning compensation lens 34A is a lens including lens components each having a predetermined refractive power according to respective distance from the optical axis, and is formed with rotational symmetry about the optical axis such that each of the lens components gives a different focal length.

Specifically, the scanning compensation lens 34A is an example formed by a single lens that converges light from the light source 21 at three different positions. One refraction surface of the scanning compensation lens 34A is formed so as to have a predetermined curvature, and a region on the other surface thereof up to a given distance centered on the optical axis CL is configured by a lens component Lz1 having a curvature so as to have a refractive power that gives a first focal length f1. Moreover, the scanning compensation lens 34A also includes a lens component Lz2 configured by a ring-shaped band at the outer periphery of the lens component Lz1 having a curvature so as to have a refractive power that gives a second focal length f2. Furthermore, the scanning compensation lenses 34A also includes a lens component Lz3 configured by a ring-shaped band at the outer periphery of the lens component Lz2 having a curvature so as to have a refractive power that gives a third focal length f3. The scanning compensation lens 34A may be set with focal lengths that are longer in the sequence from the first focal length f1 to the third focal length f3 (for example, f1 < f2 < f3), may be set with focal lengths that are shorter in this sequence (for example, f1 > f2 > f3), or may be set with focal lengths in any freely selected sequence (for example, f1 < f3 > f2).

Boundary portions to each of the lens components Lz1, Lz2, Lz3 may be formed by end portions of curvatures having refractive powers to respectively give the first focal length f1 to the third focal length f3, or may be formed by connecting end portions of curvatures such that the end portions of curvatures are a continuum. Moreover, the end portions of curvatures, or peripheries thereof, may be formed by connecting with aspherical surfaces such that the respective end portions of curvatures are a continuum.

Although the one refraction surface of the scanning compensation lens 34A is formed so as to have a single predetermined curvature in the above description, the present disclosure is not limited thereto. For example, both refraction surfaces of the scanning compensation lens 34A may have curvatures determined such that lens components have refractive powers to give respective focal lengths.

Moreover, although a preferable example has been described above of a single lens that converges light from the light source 21 at three different positions as the scanning compensation lens 34A, there is no limitation to there being three convergence positions for converging light, and there may be any number of two or more thereof. As the scanning compensation lens 34A, by converging light from the light source 21 at three different positions, for example, converging positions can be respectively set for some light and for other light outside the scanning center, with reference to the scanning center of the optical scanner 25. This means that, compared to a configuration in which aberration correction is only performed on light for the scanning center of the optical scanner 25, the scanning compensation lens 34A is able to perform aberration correction at the periphery thereof, namely according to scan angle.

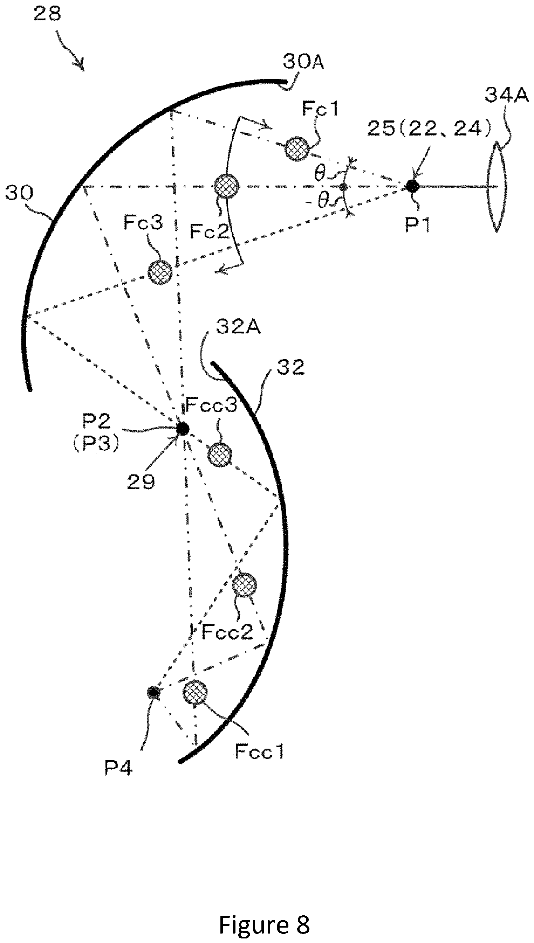

Next, description follows regarding aberration correction according to scan angle by the scanning compensation lens 34A, with reference to Figure 8. The scanning compensation lens 34A is positioned on the light path LL (Figure 6) between the light source 21 and the optical scanner 25, and converges or focuses light from the light source 21 at three different converging positions Fc1, Fc2, Fc3. These converging positions Fc1, Fc2, Fc3 correspond to the lens components Lz1, Lz2, Lz3 of the scanning compensation lens 34A. Specifically, the converging position Fc1 is a converging position converged by the lens component having the first focal length f1 of the lens component Lz1. Similarly, the converging positions Fc2, Fc3 are converging positions converged by the respective lens components having the second focal length f2 of the lens component Lz2, and the third focal length f3 of the lens component Lz3. With regard to the converging positions of light by the scanning compensation lens 34A, with reference to the scanning center of the optical scanner 25, the converging position for the scanning center thereof is the converging position Fc2. Moreover, the converging positions for some scan angles is converging position Fc1, and the converging positions for some other scan angles is converging position Fc3.

The scanning compensation lens 34A converges light from the light source 21 at the plurality of converging positions Fc1, Fc2, Fc3, and this light is scanned by the optical scanner 25 at the pupil conjugate position P1 and guided to the first reflection mirror 30. The light scanned by the optical scanner 25 is reflected at the first reflection mirror 30, and guided to the optical scanner 29. The optical scanner 29 is positioned at the pupil conjugate positions P2, P3, scans the incident light in a direction intersecting with the scan direction of the optical scanner 25 and guides the light to the second reflection mirror 32. The light scanned by the optical scanner 29 is reflected at the second reflection mirror 32, and is guided to the examined eye pupil position P4.

However, when scanning with the optical scanner 25, due to the light from the light source 21 being converged at a plurality of converging positions (focal positions) by the scanning compensation lens 34A, some light beams for each of the respective scan angles of the optical scanner are supplied as parallel light to the position of the examined eye pupil. Specifically, for the positions where light is converged by the scanning compensation lens 34A, with reference to the scanning center of the optical scanner 25, the converging position for the scanning center is the converging position Fc2. The first reflection mirror 30 guides the light converged at the converging position Fc2 by reflection so as to be converged at the converging position Fcc2 on the light incident side of the second reflection mirror 32. The second reflection mirror 32 guides the light converged at the converging position Fcc2 by reflection to the examined eye as parallel light. The converging position Fc2, the converging position Fcc2, and the lens component (for example, the lens component Lz2) can be determined from the curvature of the first reflection mirror 30 and the second reflection mirror 32 at a particular scan angle (in this case the scanning center). Note that from out of light beams passing through the scanning center of the optical scanner 25, light beams passing through the lens components Lz1, Lz3 are reflected by the first reflection mirror 30 and the second reflection mirror 32, and guided to the examined eye as either diffuse light or converging light. From out of the light beams passing through the scanning center, the light beams passing through Lz2 are condensed onto the retina, and the light beams passing through Lz1 and Lz3 are not condensed onto the retina. From out of light reflected by the retina, the light beams passing through the lens component Lz2 pass through the apertures in the light restricting elements 36 and are guided to the respective detectors (for example, the light detectors of the detection optical system on the light detector side of the SLO unit 18). From out of the light reflected at the retina, the light beams passing through the lens components Lz1 or Lz3 are not guided to the detectors due to the light restricting elements 36 because the light beams passing through the lens components Lz1 and/or Lz3 are out of focus when the optical scanner 25 is at the scanning center position.

However, when light having the same light path length as the scanning center is converged when scanning on one side with respect to the scanning center of the optical scanner 25 (scanning at a scan angle θ in the diagram), then this is supplied as diffuse light to the examined eye. Thus in the present disclosure, due to the scanning compensation lens 34A, from out of the plural converging positions of light from the light source 21, the light converged at the converging position Fc1 is shifted a specific distance to the upstream side (the pupil conjugate position P1 side) of the converging position Fc2 and is light subject to one-side scanning (scanning at the scan angle θ in the diagram). The first reflection mirror 30 accordingly converges this light at the converging position Fcc1 on the light incident side of the second reflection mirror 32, and the second reflection mirror 32 guides the light converged at the converging position Fcc1 by reflection to the examined eye as parallel light. The converging position Fc1, the converging position Fcc1, and the lens component Lz1 can be determined from the curvature of the first reflection mirror 30 and the second reflection mirror 32 at a particular scan angle. Note that light beams that pass through the lens components Lz2, Lz3 are reflected by the first reflection mirror 30 and the second reflection mirror 32, and are guided to the examined eye either as diffuse light or converging light. The light beams passing through the Lz1 are condensed onto the retina, and the light beams passing through the Lz2 and the Lz3 are not condensed onto the retina. From out of the light reflected at the retina, the light beams that have passed through the lens component Lz1 pass through the apertures in the light restricting elements 36 and are guided to the respective detectors. From out of light reflected by the retina, the light beams that have passed through the lens component Lz2 or Lz3 are not guided to the detectors due to the light restricting elements 36 because the light beams passing through the lens components Lz2 and/or Lz3 are out of focus when the optical scanner 25 is scanning at angle θ.

Moreover, when light having the same light path length as the scanning center is converged when scanning at the other side with respect to the scanning center of the optical scanner 25 (scanning at a scan angle -θ in the diagram), converging light is supplied to the examined eye. Thus in the present disclosure, due to the scanning compensation lens 34A, from out of the plural converging positions of light from the light source 21, the light converged at the converging position Fc3 is shifted a specific distance to the downstream side (the first reflection mirror 30 side) of the converging position Fc2 and is light subject to other-side scanning (scanning at scan angle -θ in the diagram). This means that the first reflection mirror 30 converges light at the converging position Fcc3 on the light incident side of the second reflection mirror 32, and the second reflection mirror 32 guides the light converged at the converging position Fcc3 by reflection to the examined eye as parallel light. The converging position Fc3, the converging position Fcc3, and the lens component Lz3 can be determined from the curvature of the first reflection mirror 30 and the second reflection mirror 32 at a particular scan angle. Note that light beams that pass through the lens components Lz1, Lz2 are reflected by the first reflection mirror 30 and the second reflection mirror 32, and are guided to the examined eye either as diffuse light or converging light. The light beams passing through the Lz3 are condensed onto the retina, and the light beams passing through the Lz1 and the Lz2 are not condensed onto the retina. From out of light reflected at the retina, light beams that have passed through the lens component Lz3 are guided to detectors through the apertures in the light restricting elements 36. From out of light reflected at the retina, light beams that have passed through the lens component Lz1 or Lz2 are not guided to the detectors due to the light restricting elements 36 because the light beams passing through the lens components Lz1 and/or Lz2 are out of focus when the optical scanner 25 is scanning at angle -θ.

As described above, the scanning compensation lens 34A is arranged on the light path before scanning of light from the light source 21, and by converging (focusing) light at a plurality of different positions simultaneously, an aberration according to scan angle that arises in the light reflected by the first reflection mirror 30 and the second reflection mirror 32 can be corrected or suppressed.

Next, description follows regarding another example of the scanning compensation lens 34. A scanning compensation lens 34B illustrated in Figure 7 includes a plurality of lens components each giving predetermined focal lengths, and converges light at different positions with each of the lens regions. Specifically, the scanning compensation lens 34B is an example of a lens configured by one lens component and another lens component, when the lens is cut in a sagittal direction cross-section including the optical axis of light from the light source 21, giving focal lengths that are different focal lengths. The scanning compensation lens 34B has one refraction surface formed so as to have a predetermined curvature, and on the other surface thereof, one lens component Lh1 has a curvature, when the lens is cut in a sagittal direction cross-section including the optical axis CL, to give a refractive power of a first focal length f1. The scanning compensation lens 34B also includes another lens component Lh2 having a curvature, when the lens is cut in a sagittal direction cross-section including the optical axis CL, giving a refractive power of a second focal length f2. The scanning compensation lens 34B is an example of a lens in which the first focal length f1 is shorter than the second focal length f2 (f1 > f2). Note that a lens component Lh3 present at the remaining portion of the one refraction surface of predetermined curvature may be configured as a lens component with a curvature having a refractive power of a third focal length f3. This means that lens configuration can be simplified in the scanning compensation lens 34B compared to the scanning compensation lens 34A, enabling a simplification of manufacturing to be achieved.

A scanning compensation lens 34C illustrated in Figure 7 is a diffraction lens. For example, a diffraction lens may be applied as the scanning compensation lens 34C to utilize the principle of diffraction to distributed light energy so that incident light crosses at focal points at plural different positions. Namely, the scanning compensation lens 34C is an example of a diffraction lens formed so as to converge light from the light source 21 at three different positions simultaneously. The scanning compensation lens 34C includes three diffraction step regions that have the same step height and the same curvature. Specifically, the scanning compensation lens 34C is formed with ring-band shaped diffraction step regions Ls1, Ls2, Ls3 that distribute light such that light crosses at three focal points, the first focal length f1, the second focal length f2, and the third focal length f3, as described above. By utilizing the principle of diffraction, the scanning compensation lens 34C is able to form a lens capable of distributing more light energy to each of the plural focal positions than the scanning compensation lens 34A. Note that the scanning compensation lens 34C, which is a diffraction lens, may be configured so as to also include a refraction lens. This accordingly means that, for example, the scanning compensation lens 34C is applicable to a refraction-diffraction lens including a full-surface diffraction multi-focal lens with a diffraction step region and a refraction region.

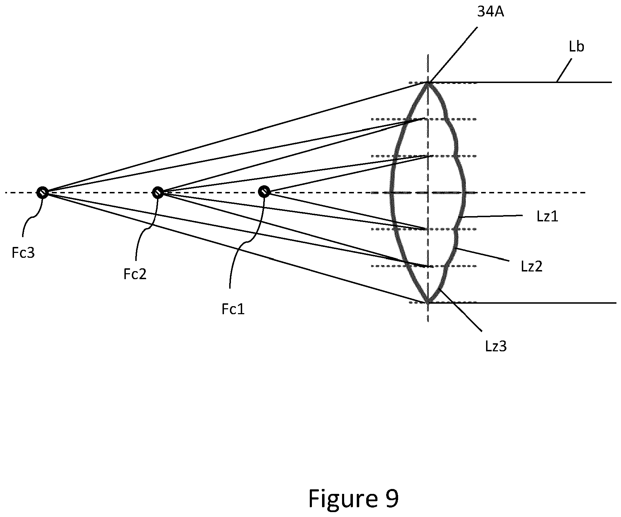

Figure 9 is a schematic illustration of the light beam Lb passing through the scanning compensation lens 34A. The optical scanner has been omitted from Figure 9 for clarity. However, as described above the focal positions Fc1, Fc2 and Fc3 may be located on the beam path between the optical scanner and the mirror. As shown in Figure 9 the light beam Lb irradiates the optical element 34A such that the light beam Lb passes through multiple regions of the scanning compensation lens 34A and respective portions of the light beam are focused simultaneously at focal positions Fc1, Fc2 and Fc3 by the lens components Lz1, Lz2, Lz3 respectively. The optical element 34A is dimensioned such that the majority or all of the optical element is illuminated by the light beam 34A such that the optical element 34A focuses all of the input light beam 34A at a respective focal position Fc1, Fc2 and Fc3. The skilled reader will understand that whilst the example optical element 34A focuses the light beam at three focal positions simultaneously in another embodiment the optical element may focus the beam of light Lb at two or more focal points simultaneously.

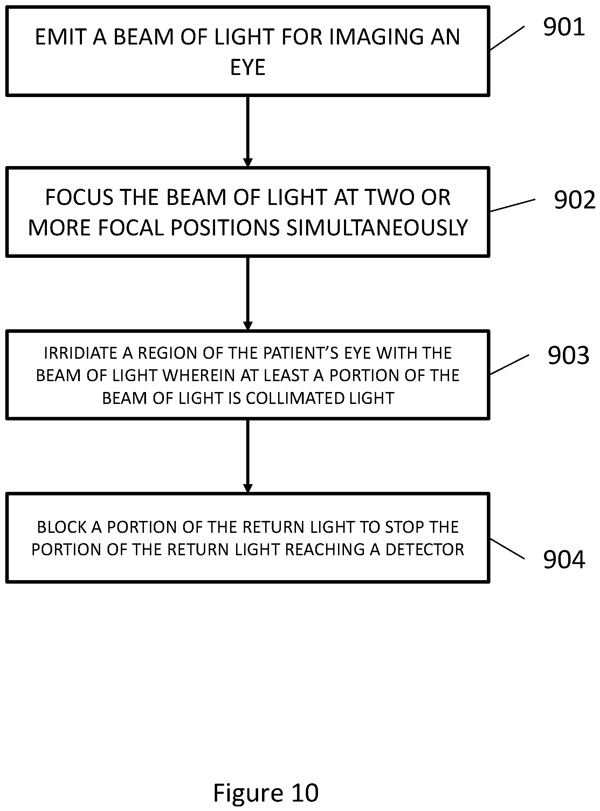

Figure 10 shows a flow chart outlining a method of correcting aberrations in images acquired by an ophthalmic imaging device such as a wide-field or ultra-wide field scanning laser ophthalmoscope. In Step 901 a light source is operated to emit a beam of light for imaging a patient's eye 12. The beam of light may be a laser beam emitted from the light source 21.

In Step 902 the beam of light is focused at two or more focal positions simultaneously. Focusing the beam of light at two or more focal positions simultaneously beneficially suppresses or corrects aberrations in the beam of light that may be imparted on the beam of light by one or more elliptical mirrors within the ophthalmic imaging device as described above. For example, each of the two or more focal positions may correspond to an aberration that the one or more elliptical mirrors impart on the beam of light at a respective scan angle of the scanner. Focusing the beam of light at two or more focal positions simultaneously may comprise focusing a first portion of the light beam at a first focal position and simultaneously focusing a second portion of the light beam at a second focal position.

Focusing the beam of light may be performed at a location in the ophthalmic imaging device where the beam of light is stationary. For example, where the beam of light is stationary in space such that the beam path of the beam of light does not vary temporally. The location of the focusing in the ophthalmic imaging device may be a location between the light source and the scanner such that the beam of light is stationary.

In Step 903 a region of the patient's eye is illuminated with the beam of light. Illuminating the region of the patient's eye with the beam of light may comprise illuminating the patient's eye with a portion of the light beam that is collimated light and a portion of the light beam that is non-collimated light (for example converging or diverging light). The portion of the light beam that is collimated light will be focused on the patient's retina by the patient's eye 12. The non-collimated light is not focused on the retina and as such the non-collimated light reflected by the retina is similarly unfocused light. The aberration in the beam of light may be a de-focus aberration imparted on the beam of light by the one or more elliptical mirrors at a respective scan angle and the focal position may be a position that compensates for the de-focus aberration imparted on the beam of light by the elliptical mirror such that at least a portion of the beam of light is substantially parallel light that is delivered to the patient's eye.

In Step 904 the light reflected by the patient's retina that is not focused is discarded or blocked prior to reaching a detector in the ophthalmic imaging device. Discarding the portion of the return light that is not focused may comprise passing the return light through an aperture that is located at a position that is conjugate to the retina. Light reflected by the patient's retina that is focused can pass through the aperture and light that is not focused on the retina is similarly not focused on the conjugate position where the aperture is located and as such is blocked by the aperture. Beneficially only light that was focused on the patient's retina is transmitted to the detector thereby removing de-focus aberrations from the return light that is detected by the detector.

Note that the description above is of a case in which, as the scanning compensation lens 34, an optical lens is formed in which refractive power or power of diffraction is distributed centered on light at the scanning center of the optical scanner 25. However, the above configuration is merely an example thereof, and the scanning compensation lens 34 may have any combination of refractive power and/or power of diffraction such that each light beam scanned by the optical scanner 25 is emitted as parallel light to the examined eye.

Moreover, the above scanning compensation lens 34 (for example, 34A, 34B, 34C) is not limited to compensating for all scan angles. For example, the scanning compensation lens 34 may be configured as a lens or optical system so as to compensate at least some scan angles of the optical scanner 25. Namely, although in the above example the light scanned by the optical scanners 25, 29 was converged at plural different positions, the present disclosure is not limited thereto. Namely, the scanning compensation lens 34 may be configured so as to converge incident light at a predetermined plural number of positions corresponding to at least some plural scan angles of the light scan angles of the optical scanner 25, so as to be illuminated as parallel light onto the examined eye. Adopting such an approach enables the scan angles of the optical scanner 25 to be converged at plural different positions to be selected.

Moreover, the scanning compensation lens 34 described above may be configured so as to converge incident light such that a converging position where light at the scanning center of the optical scanner 25 is converged, and a converging position where light other than at the scanning center is converged, are different positions. Namely, the converging position of light other than at the scanning center may be made different with reference to the scanning center of the optical scanner 25. Configuring with reference to the scanning center enables simple adjusting of the plural converging positions to be converged at.

Moreover, as described above, applying the scanning compensation lens 34 to a lens having a predetermined refractive power according to distance from the optical axis, and formed with rotational symmetry about the optical axis so as to give plural focal lengths, enables simple manufacture of the scanning compensation lens 34.

Note that although a case is described above in which the scanning compensation lens 34 is arranged on the light path before scanning of light from the light source 21, this may be combined with a configuration arranged on the light path after scanning of light from the light source 21. For example, aberration correction may be performed by arranging the scanning compensation lens 34 on the light path before scanning, and arranging a correction lens serving as a correction element of the present disclosure on the light path after scanning. For example, a correction lens may be arranged for scan angles in a predetermined range. In such cases, the correction lens can function as a lens to supplement aberration correction by the scanning compensation lens 34. In this manner, due to the arrangement of the correction lens, aberration correction can be performed corresponding to scan angles in a predetermined range.

Although description above is of a case in which a single lens or lens system is applied as the scanning compensation lens 34, the present disclosure is not limited to an optical element including lens components centered on an optical axis. For example, an optical element including a region of a light transmitting component having a refractive power giving a different focal length to elsewhere may be applied for at least a portion of through-pass light. Namely, application may be made to a lens or an optical element having plural refraction regions with predetermined respective refractive powers so as to give plural focal lengths. In this manner, an optical element may be formed in which a refraction region can be specified according to scan angle.

Moreover, although as a scanning compensation lens 34 an example has been described above for a lens giving two or three focal lengths in which the refractive power is changed stepwise, a configuration may be adopted including an optical system with an optical component including an aspherical surface with refractive power that changes as a continuum, such that the focal length changes as a continuum.

Note that although an example has been described above in which the scanning compensation lens 34 is arranged on a light path before scanning of light from the light source 21, the scanning compensation lens 34 may be attached to a device at the periphery of an optical element in the imaging optical system 19 including the wide-angle optical system 28. For example, the scanning compensation lens 34 may be attached to a window letting light in and out provided to a protection case for protecting the optical scanner 25 (22, 24) that is a polygon mirror, a mirror galvanometer, or the like.

In the exemplary embodiment described above, although the image processing (Figure 5) is executed by the server 140, the present disclosure is not limited thereto, and the image processing may be executed by the ophthalmic device 110, the viewer 150, or by an additional image processing device additionally provided on the network 130. Moreover, in the present disclosure, unless this results in inconsistencies, there may be one of each of the configuration elements (devices etc.), or two or more present thereof.

Although explanation has been given in the exemplary embodiments described above regarding an example in which image processing is implemented by a software configuration using a computer, the technology disclosed herein is not limited thereto. For example, instead of a software configuration using a computer, the image processing may be executed solely by a hardware configuration such as a field programmable gate array (FPGA) or an application specific integrated circuit (ASIC). Alternatively, a configuration may be adopted in which some processing out of the image processing is executed by a software configuration, and the remaining processing is executed by a hardware configuration.

In order to execute the above processing using a computer, a program written with computer executable code for the above processing may be stored and distributed on a storage medium such as an optical disc or the like.

Moreover, the above operations of the processor are not limited to being performed by a single processor, may be performed by plural processors collaborating with each other, and may be performed by cooperation between plural processors present at physically separated locations.

All publications, patent applications and technical standards mentioned in the present specification are incorporated by reference in the present specification to the same extent as if each individual publication, patent application, or technical standard was specifically and individually indicated to be incorporated by reference.

## Claims

1. An ophthalmic imaging device (110) for imaging a patient's eye (12), the ophthalmic imaging device (110) comprising:
a light source (21) arranged to emit a beam of light (Lb) along a light path;
a scanner (25) configured to scan the beam of light (Lb) emitted from the light source (21);
an elliptical mirror (30, 32) comprising a first focal point and a second focal point, wherein the scanner (25) is arranged to scan the beam of light (Lb) across a region of the patient's eye (12) via the first focal point; and
an optical element (34) located on the light path between the light source (21) and the scanner (25), wherein the optical element (34) is configured to focus a first portion of the beam of light (Lb) at a first focal position (Fc1) on the light path, and to simultaneously focus a second portion of the beam of light at a second focal position (Fc2) on the light path, wherein the first focal position (Fc1) is located at a different position to the second focal position (Fc2) on the light path.

2. The ophthalmic imaging device (110) of Claim 1, wherein the first and second focal positions (Fc1, Fc2) are located on the light path between the scanner (25) and the elliptical mirror (30).

3. The ophthalmic imaging device (110) of Claim 1 or Claim 2, wherein the first and second focal positions (Fc1, Fc2) each correspond to an aberration that the elliptical mirror (30) imparts on the beam of light (Lb) at respective first and second scan angles of the beam of light (Lb) such that at least a portion of light delivered to the patient's eye (12) is substantially collimated light when the scanner (25) is at the first and second scan angles.

4. The ophthalmic imaging device (110) of any one of Claims 1 to 3, wherein the optical element (34) comprises a first region (Lz1) having a first refractive power and a second region (Lz2) having a second refractive power and wherein light passing through the first region (Lz1) of the optical element (34) is focused to the first focal position (Fc1) and light passing through the second region (Lz2) of the optical element (34) is focused to the second focal position (Fc2).

5. The ophthalmic imaging device (110) of any one of Claim 1 to Claim 4, wherein the optical element (34) is a lens formed with rotational symmetry about an optical axis of the lens.

6. The ophthalmic imaging device (110) of any one of Claim 1 to Claim 5, wherein the optical element (34) is a lens comprising a plurality of refraction regions each having a predetermined refractive power so as to provide a plurality of focal lengths for focusing the beam of light (Lb) incident on the optical element (34).

7. The ophthalmic imaging device (110) of any one of Claim 1 to Claim 6, further comprising an aperture (36) located at a position that is conjugate with a pupil of the patient's eye, wherein the aperture (36) is arranged to transmit focused light that has been reflected by the patient's eye (12).

8. The ophthalmic imaging device (110) of any one of Claim 1 to Claim 7, further comprising a second elliptical mirror (32).

9. The ophthalmic imaging device (110) of any one of Claim 1 to Claim 8, further comprising a correction element that is arranged on a light path between the scanner (25) and the patient's eye (12), and that is configured to correct incident light such that light scanned by the scanner (25) is irradiated onto the patient's eye (12) as substantially parallel light.

10. The ophthalmic imaging device (110) of any one of Claim 1 to Claim 9, wherein the optical element (34) is dimensioned such that the beam of light (Lb) illuminates the majority of the optical element (34) and such that a major portion of light reflected by the region of the patient's eye (12) does not pass through the optical element (34).

11. A method of correcting aberrations in an ophthalmic imaging device (110) for imaging a patient's eye (12), the ophthalmic imaging device (110) comprising an elliptical mirror (30) and a scanner (25), the method comprising:
emitting a beam of light (Lb) along a light path;
focusing the beam of light (Lb) at two or more focal positions (Fc1, Fc2) simultaneously to compensate for an aberration that the elliptical mirror (30) imparts on the beam of light (Lb) when the beam of light (Lb) is incident on the elliptical mirror (30), wherein focusing the beam of light (Lb) comprises focusing a first portion of the beam of light (Lb) at a first focal position (Fc1) on the light path, and simultaneously focusing a second portion of the beam of light at a second focal position (Fc2) on the light path,
wherein the first focal position (Fc1) is located at a different position to the second focal position (Fc2) on the light path; and
scanning the beam of light (Lb) across a region of the patient's eye (12) via the elliptical mirror (30).

12. The method of Claim 11, wherein each of the two or more focal positions (Fc1, Fc2) correspond to an aberration that the elliptical mirror (30) imparts on the beam of light (Lb) at respective scan angles of the scanner (25).

13. The method of Claim 11 or Claim 12, wherein focusing the beam of light (Lb) is performed at a location in the ophthalmic imaging device (110) where the beam of light (Lb) is stationary.

14. The method of any one of Claims 11 to 13, comprising irradiating the patient's eye (12) with the beam of light (Lb) wherein a first portion of the beam of light is substantially collimated light and wherein a second portion of the beam of light is converging or diverging light.

15. The method of Claim 14, comprising blocking the second portion of the beam of light after the second portion of the beam of light has been reflected by the patient's eye to prevent the second portion of the beam of light irradiating a detector.
